# EUROPEAN PATENT APPLICATION

(11) **EP 4 710 930 A1**
(43) Date of publication of application: **18.03.2026**
(21) Application number: 24823608.5
(22) Date of filing: 28.05.2024
(51) Int. Cl.: A61K 31/723, A61K 31/737, A61K 9/00, A61P 31/16

(54) **ANTI-INFLUENZA VIRUS COMPOSITION CONTAINING XANTHAN GUM OR FUCOIDAN**

(30) Priority: 12.06.2023 KR 20230075141
(71) Applicant: Daewoong Pharmaceutical Co., Ltd., Hwaseong-si, Gyenoggi-do 18623 (KR)
(72) Inventor: LEE, Jae Won, Yongin-si, Gyeonggi-do 17028 (KR); NA, Yu Jeong, Yongin-si, Gyeonggi-do 17028 (KR); LEE, Dong Jin, Yongin-si, Gyeonggi-do 17028 (KR); CHOI, Jong Seo, Yongin-si, Gyeonggi-do 17028 (KR); LEE, Kyung Min, Yongin-si, Gyeonggi-do 17028 (KR); KIM, Gwan Young, Yongin-si, Gyeonggi-do 17028 (KR)
(74) Representative: Stolmár & Partner Patentanwälte PartG mbB
(86) International application number: PCT/KR2024/007193
(87) International publication number: WO 2024/258087

(57) **Abstract**

The present invention relates to an antiviral composition against influenza virus (Influenza virus), comprising xanthan gum or fucoidan as an active ingredient, a pharmaceutical composition for the prevention or treatment of influenza virus infection, and a method for preventing or treating influenza virus infection using the same.

## Description

### TECHNICAL FIELD

The present invention relates to an antiviral composition against influenza virus (Influenza virus) comprising, as an active ingredient, xanthan gum or fucoidan, a pharmaceutical composition for the prevention or treatment of influenza virus infection, and a method for preventing or treating influenza virus infection using the same.

### BACKGROUND ART

The influenza virus belongs to the orthomyxoviruses and is a representative pathogenic virus that claims thousands to tens of thousands of lives every season. Since the 20th century, influenza has crossed species barriers and undergone variation, thereby driving epidemic colds and being a virus having such a high mutation rate that it can render already known drugs meaningless. This disease, which is commonly referred to as epidemic influenza, generally causes chills, fever, sore throat, myalgia, cough, lethargy and malaise, and although such nonspecific symptoms are only more severe than those of an ordinary cold and are not, by themselves, fatal to the extent of threatening life, they can cause fatal complications when accompanied by pneumonia or Reye's syndrome. In general, respiratory infection mainly occurs via the cough, sputum or sneezing of an infected person, and many variants arise when infection is transmitted to humans from other animals (particularly birds). There are three types of influenza virus: influenza virus A, influenza virus B, and influenza virus C.

Among these, the influenza that caused a pandemic through interspecies transmission and that was prevalent in 2009, the so-called new flu, is of the influenza A H1N1 type, which first broke out in Mexico and, with the emergence of various variants, spread to many countries, and the World Health Organization (WHO) defined this as a "global pandemic."

In the case of influenza, it is generally common to employ preventive defensive treatment by administering a purified inactivated strain before the influenza season, but, for patients whose symptoms have already appeared during the influenza season, prevention is not possible, and therefore the symptoms must be alleviated by a therapeutic approach. In the case of "Tamiflu," which halted the spread of the new flu that had been spreading uncontrollably, although it is excellent in terms of effectiveness, cases of adverse side effects such as the occurrence of hallucinations after ingestion have from time to time been reported in the media.

Drugs conventionally used for the treatment of influenza include oseltamivir (trade name: Tamiflu), zanamivir (trade name: Relenza), peramivir (Peramivir), and amantadine (Amantadine).

Oseltamivir, known as Tamiflu, is currently mainly used for the treatment of H1N1 influenza A. Tamiflu is the only avian influenza (AI) therapeutic agent that is exclusively produced in the world.

It is an antiviral agent that exerts a therapeutic effect by inhibiting the function of an enzyme that propagates the virus, and exhibits a great effect when administered within 48 hours after the onset of symptoms.

Its main therapeutic effects include reducing the worsening of influenza symptoms, reducing the occurrence of secondary complications such as bronchitis or pneumonia, and shortening the incubation period of influenza. It is also used as a therapeutic agent for influenza A and B.

Zanamivir has the trade name Relenza. It acts as a neuraminidase inhibitor and is used for the treatment of influenza A and B.

However, oseltamivir has the side effect that severe vomiting symptoms may occur, and although zanamivir has a high antiviral effect, it has the disadvantages of low bioavailability and rapid excretion via the kidneys.

Against this background, there is a continuous demand for the development of antiviral agents having excellent infection-inhibitory and therapeutic effects.

### DESCRIPTION OF EMBODIMENTS

### TECHNICAL PROBLEM

The technical problem to be solved by the present invention is to provide an antiviral composition against influenza virus, comprising xanthan gum or fucoidan as an active ingredient.

Another technical problem to be solved by the present invention is to provide a pharmaceutical composition for the prevention or treatment of a viral infectious disease caused by influenza virus, comprising xanthan gum or fucoidan as an active ingredient.

A further technical problem to be solved by the present invention is to provide a method for preventing or treating influenza virus infection, the method comprising a step of administering the pharmaceutical composition to a subject.

A still further technical problem to be solved by the present invention is to provide the use of a composition comprising xanthan gum or fucoidan as an active ingredient for preventing or treating influenza virus infection.

A yet further technical problem to be solved by the present invention is to provide the use of a composition comprising xanthan gum or fucoidan as an active ingredient for the manufacture of a medicament for treating influenza virus infection.

### TECHNICAL SOLUTION

According to an aspect of the present invention, there is provided an antiviral composition against influenza virus (Influenza virus), comprising xanthan gum or fucoidan as an active ingredient.

In the present invention, the term "xanthan gum" refers to a polysaccharide derived from a natural product, has low toxicity, and is an exopolysaccharide produced by fermentation of *Xanthomonas campestris,* a bacterium of the genus *Xanthomonas* that causes bacterial blight of beans. An aqueous solution of xanthan gum has a high viscosity at a low concentration and has the characteristic that its viscosity is maintained almost constant over a wide temperature range (10-70 °C), pH (pH 6-9), and electrolyte concentration. Due to such rheological properties, xanthan gum is used in industries such as food and pharmaceuticals as a stabilizer, thickener, and suspending agent for suspensions.

In the present invention, the "Fucoidan" is a polysaccharide which is a type of watersoluble dietary fiber and is contained in brown algae such as sea mustard and kelp. Depending on the harvesting time and species of the brown algae, the average molecular weight of fucoidan varies from 100,000 to 2,000,000 Da, and the fucose content and sulfate group content, which have an important influence on the physiological activity of fucoidan, also differ.

In the present invention, the "Influenza virus" has, on its surface, two surface antigens, namely the glycoproteins hemagglutinin (HA), neuraminidase (NA), and has eight segmented RNAs present in the interior thereof. HA binds to sialic acid residues present on the surface of a host cell, thereby allowing the virus to attach to and penetrate into the host cell. NA plays a role in inducing viruses that have completed proliferation in an infected host cell to proliferate into other host cells by cleaving an α-ketosidic bond between a disaccharide portion on the cell surface and a neuraminic acid residue. An in vivo defense mechanism against viruses acts in such a manner that it recognizes HA to interfere with the binding of the virus to the host cell, or binds to NA to inhibit the proliferation of viruses within an infected host cell into adjacent cells. However, through various combinations of 16 types of HA and 9 types of NA, the virus generates numerous possible mutations and thereby acquires resistance to the defense mechanism.

In one embodiment, the influenza virus may be an influenza A virus, and the influenza A virus may be of the H1N1 type, but is not limited thereto.

In the present invention, the term "antiviral" means all mechanisms and activities that inhibit various subtypes and variant virus particles of a virus from entering host cells, or that inhibit virus particles from replicating or proliferating within host cells after infection.

In one embodiment, the composition may be used for immune measures against an endemic, prepandemic, or pandemic caused by influenza virus.

The term "immunization" refers to the induction of immunity for the prevention of infectious diseases, and includes all methods of enhancing or acquiring immunity in a living body.

According to another aspect of the present invention, there is provided a pharmaceutical composition for the prevention or treatment of a viral infectious disease caused by influenza virus, comprising xanthan gum or fucoidan as an active ingredient.

In the present invention, the term "influenza virus infection" means that the lung and/or airway is infected by one of the influenza viruses. It is accompanied by a respiratory disease, and when the inflammatory response is aggravated, it can lead to tissue damage.

In one embodiment of the present invention, it was confirmed that administration of xanthan gum and/or fucoidan exhibited an alleviating effect on inflammation and tissue damage caused by influenza virus infection.

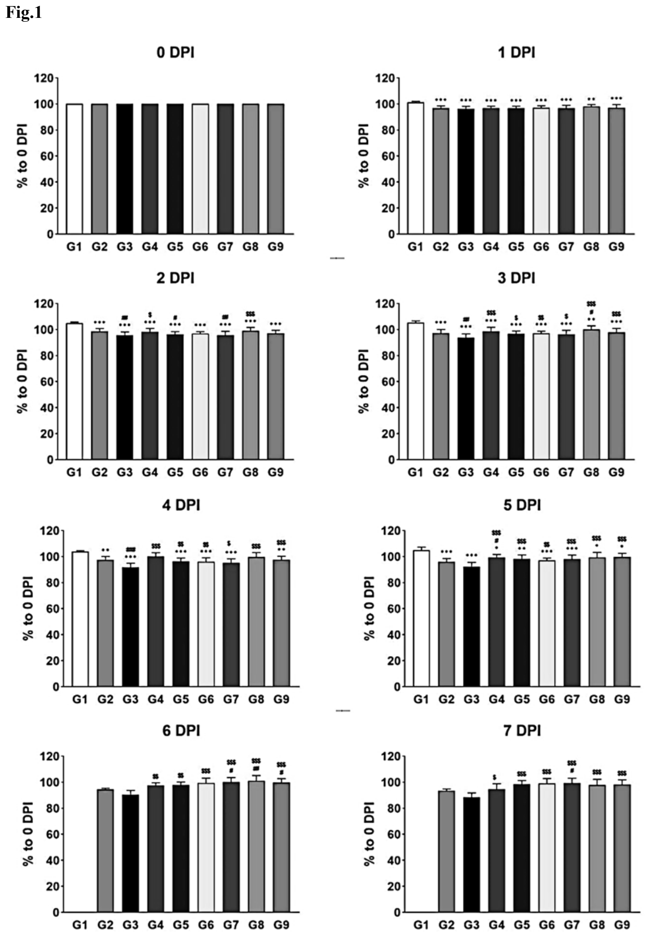

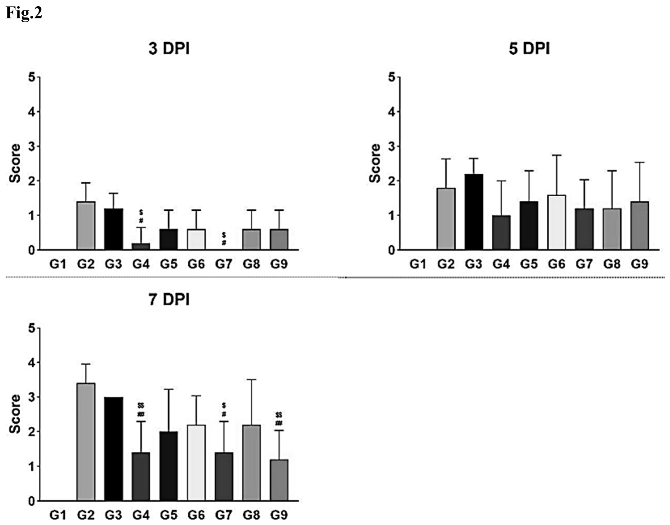

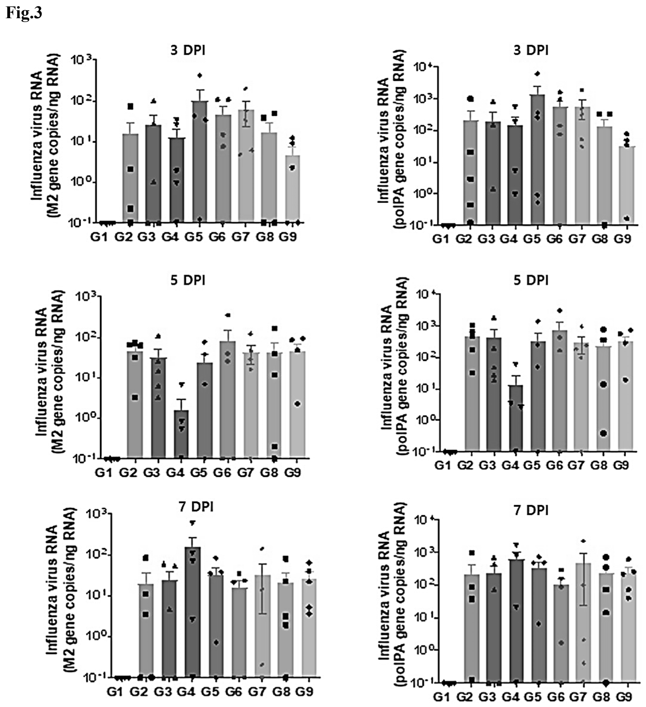

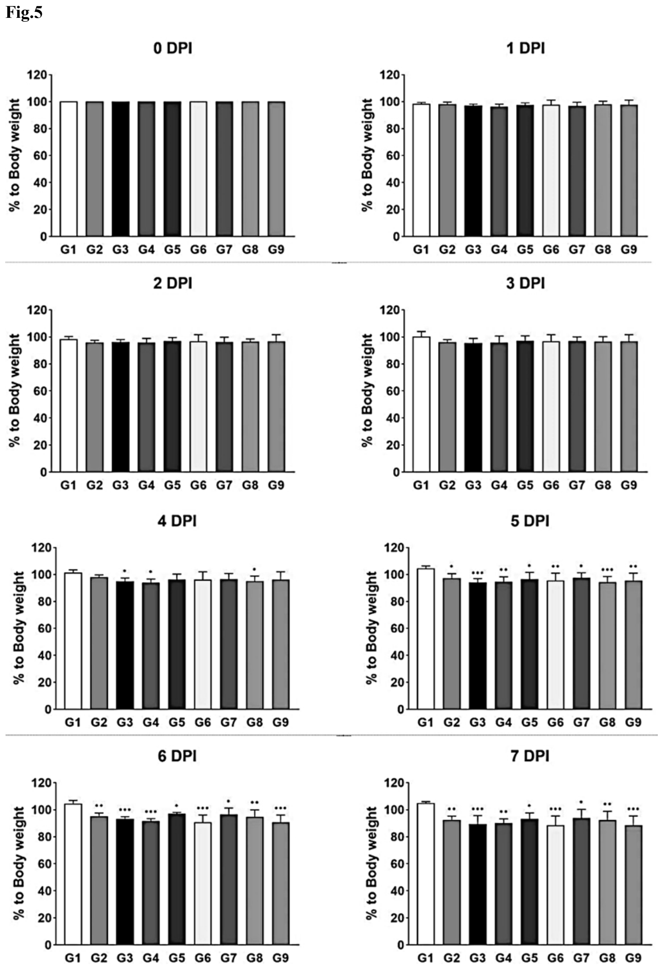

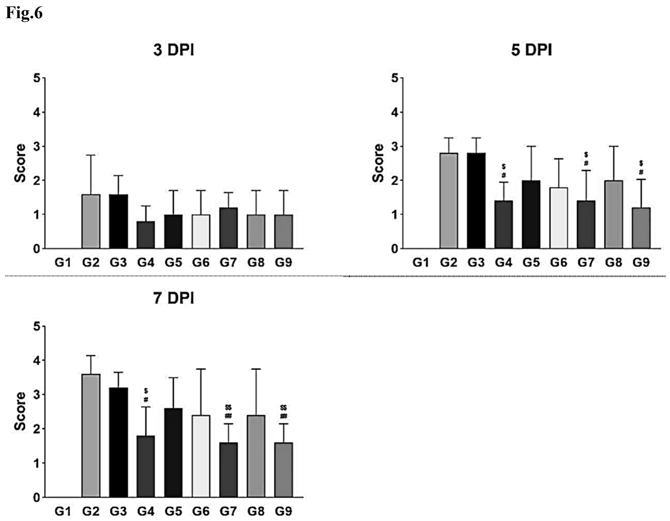

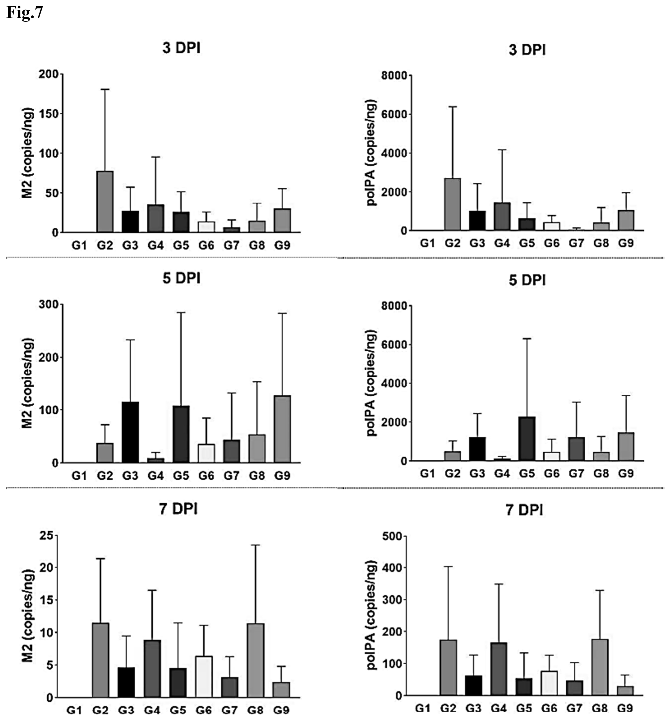

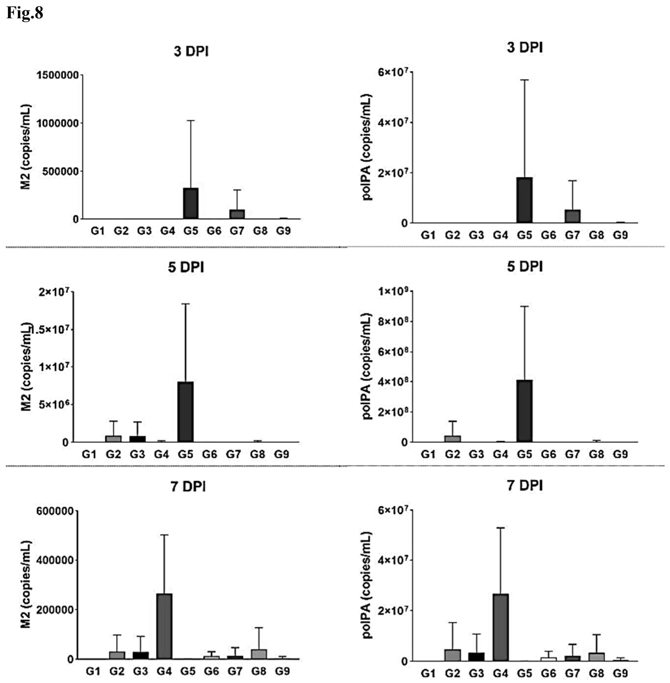

In one embodiment, when administered before and/or after viral infection, one of the symptoms of influenza virus infection, namely weight loss, was alleviated (Table 3, Table 8, FIG. 1 and FIG. 5), infiltration of inflammatory cells was reduced (Table 5, Table 9, FIG. 2 and FIG. 6), and it was also confirmed that the viral titer in the lung and nasal cavity was decreased (lung: Table 10, Table 11, FIG. 3 and FIG. 7; nasal cavity: Table 12, Table 13, FIG. 3 and FIG. 8).

The composition of the present invention, when administered for prophylactic or therapeutic purposes, can exhibit an inhibitory effect against influenza virus while also alleviating inflammation and reducing damage in lung tissue. In particular, in the case of a highly virulent influenza virus, if the anti-inflammatory action fails, a large number of inflammatory cells may infiltrate the lungs and an excessive amount of inflammatory cytokines may be secreted, thereby causing a cytokine storm, and the pharmaceutical composition of the present invention as described above can effectively respond thereto.

In addition, the pharmaceutical composition of the present invention can be applied for use in the prevention or treatment of diseases that may be caused by influenza virus infection. For example, the diseases that may be caused by the influenza virus infection may be one or more selected from the group consisting of a cold, influenza, cough, sneezing, runny nose, myalgia, pharyngitis, sepsis, pneumonia, nasal cavity obstruction, laryngitis, sore throat, hoarseness, headache, pain in the paranasal sinuses, rhinitis, pharyngitis, bronchitis, asthma, fever, dyspnea, general lassitude and chills, but are not limited thereto.

In the present invention, the term "prevention" means any act by which the composition of the present invention delays the occurrence of infection caused by influenza virus.

In the present invention, the term "treatment" means any act by which the composition of the present invention improves or benefits the symptoms of infection caused by influenza virus.

In one embodiment, the pharmaceutical composition of the present invention may be in a non-oral dosage form, and more specifically may be administered intranasally or into the nasal cavity. The intranasal or nasal cavity administration may be carried out in the form of a spray, aerosol, or inhalation.

In addition, the pharmaceutical composition of the present invention can be applied in a pharmaceutically effective amount, and the term "pharmaceutically effective amount" means an amount sufficient to treat a disease at a reasonable benefit/risk ratio applicable to medical treatment. The effective dosage level may be determined depending on factors including the sex and age of the patient, the type and severity of the disease, the activity of the drug, the sensitivity to the drug, the time of administration, the route of administration and the rate of excretion, the duration of treatment, drugs used in combination, and other factors well known in the medical field.

Another aspect of the present invention relates to a kit for the prevention or treatment of influenza virus, comprising the pharmaceutical composition.

The kit of the present invention is not particularly limited in its type, and a kit in a form conventionally used in the relevant technical field may be used. The kit of the present invention may be packaged in such a manner that xanthan gum or fucoidan is contained in an individual container, or in such a manner that xanthan gum or fucoidan is contained together with another drug or excipient in a single container divided into one or more compartments, and each active ingredient may be packaged in a unit dosage form for a single administration, but is not limited thereto.

In the kit, the xanthan gum or fucoidan may be administered at an appropriate time depending on the health condition and the like of the subject to be administered, and, if necessary, may be administered in combination with an additional other drug simultaneously, sequentially, or in reverse order.

Another aspect of the present invention relates to a method for preventing or treating influenza virus infection, the method comprising a step of administering the pharmaceutical composition to a subject.

In the present invention, the term "subject" includes an animal or a human having an influenza virus infection, the symptoms of which can be improved by administration of the pharmaceutical composition according to the present invention. By administering the therapeutic composition according to the present invention to a subject, influenza virus infection can be effectively prevented and treated.

In the present invention, the term "administration" means introducing a given substance into a human or an animal by any appropriate method, and the route of administration of the therapeutic composition according to the present invention may be oral or parenteral via any conventional route, as long as it can reach the target tissue. In addition, the therapeutic composition according to the present invention may be administered by any device through which the active ingredient can move to the target cells.

The preferred dosage of the pharmaceutical composition according to the present invention may vary depending on the condition and body weight of the patient, the degree of the disease, the dosage form, the route and period of administration, and the like, but can be appropriately selected by a person skilled in the art.

Another aspect of the present invention relates to the use of the pharmaceutical composition for the treatment of influenza virus infection.

A further aspect of the present invention relates to the use of the antiviral composition for the manufacture of a medicament for treating influenza virus infection. The antiviral composition comprises xanthan gum or fucoidan as an active ingredient.

### ADVANTAGEOUS EFFECTS OF INVENTION

The antiviral composition against influenza virus according to the present invention, which comprises xanthan gum or fucoidan as an active ingredient, exhibits preventive and therapeutic effects against influenza virus and reduces the occurrence of tissue lesions and infiltration of inflammatory cells caused by influenza virus infection, and thus can be used in the development of antiviral agents for the prevention or treatment of infections caused by influenza virus.

The effect of the present invention is not limited to the aforementioned effects, and it should be understood to include all possible effects deduced from the configuration of the invention described in the detailed description or the claims of the present invention.

### BRIEF DESCRIPTION OF THE DRAWINGS

FIG. 1 shows the results of observing changes in body weight upon administration of the test substance prior to influenza virus infection.
FIG. 2 shows, in a scored form, the results of histopathological evaluation upon administration of the test substance prior to influenza virus infection.
FIG. 3 shows the results of measuring viral titer in the lung, upon administration of the test substance prior to influenza virus infection, through the copy numbers of M2 and pol PA genes.
FIG. 4 shows the results of measuring viral titer in the nasal cavity, upon administration of the test substance prior to influenza virus infection, through the copy numbers of M2 and pol PA genes.
FIG. 5 shows the results of observing changes in body weight upon administration of the test substance after influenza virus infection.
FIG. 6 shows, in a scored form, the results of histopathological evaluation upon administration of the test substance after influenza virus infection.
FIG. 7 shows the results of measuring viral titer in the lung, upon administration of the test substance after influenza virus infection, through the copy numbers of M2 and pol PA genes.
FIG. 8 shows the results of measuring viral titer in the nasal cavity, upon administration of the test substance after influenza virus infection, through the copy numbers of M2 and pol PA genes.
FIG. 9 shows the results of observing changes in body weight according to the xanthan gum content, upon administration of the test substance prior to influenza virus infection.
FIG. 10 shows, in a scored form, the results of histopathological evaluation according to the xanthan gum content, upon administration of the test substance prior to influenza virus infection.
FIG. 11 shows the results of measuring viral titer in the lung according to the xanthan gum content, upon administration of the test substance prior to influenza virus infection.
FIG. 12 shows the results of measuring viral titer in the nasal cavity according to the xanthan gum content, upon administration of the test substance prior to influenza virus infection.

### DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Preparation Example 1. Preparation of Test Substance

A test substance was prepared according to the composition shown in Table 1 below. Specifically, benzoic acid, citric acid, and sorbitol (D-sorbitol) were added to about 0.8 mL of purified water, and stirred to dissolve. Thereafter, in accordance with each embodiment composition shown in Table 1 below, each of HPMC, xanthan gum, bentonite, and fucoidan as main components was weighed and added, and the mixture was stirred for 2 hours or more to dissolve, after which purified water was added to make the total volume 1 mL.

**[Table 1]**

| **Component (mg/mL)** | **Example 1** | **Example 2** | **Example 3** | **Example 4** | **Example 5** | **Example 6** | **Example 7** |
|---|---|---|---|---|---|---|---|
| HPMC | 4.6 | - | - | - | - | - | - |
| Xanthan gum | - | 0.1 | - | - | 0.25 | 0.5 | 1 |
| Bentonite | - | - | 9 | - | - | - | - |
| Fucoidan | - | - | - | 8 | - | - | - |
| Benzoic acid | 0.6 | 0.6 | 0.6 | 0.6 | 0.6 | 0.6 | 0.6 |
| Citric acid | 0.1 | - | 0.4 | 0.3 | - | - | - |
| Sorbitol | 35 | 35 | 35 | 35 | 35 | 35 | 35 |
| Purified water | q.s. | q.s. | q.s. | q.s. | q.s. | q.s. | q.s. |
| Total volume | 1 mL | 1 mL | 1 mL | 1 mL | 1 mL | 1 mL | 1 mL |

### Comparative Example 1. Combination of Excipients

A negative control substance was prepared as a combination of excipients not containing the components HPMC, xanthan gum, bentonite, and fucoidan, by mixing benzoic acid, citric acid, sorbitol, and purified water, and the content of each excipient was made the same as the content in Table 1 above. The negative control substance prepared in this manner was used as Comparative Example 1.

### Comparative Example 2. Oseltamivir

Oseltamivir, as one of the positive control substances, was purchased from Sigma-Aldrich and used as Comparative Example 2.

Oseltamivir is marketed under the trade name Tamiflu, and, as an antiviral agent, is used to treat infectious diseases caused by influenza virus and for prophylactic purposes. In particular, it has the effect of inhibiting the H1N1 variant that causes so-called new influenza due to mutation of influenza A virus.

### Comparative Example 3. Cold Mask Nasal Spray

Cold Mask nasal spray (Hanmi Pharmaceutical Co., Ltd.) was purchased as one of the positive control substances and used as Comparative Example 3.

### Experimental Example 1. Confirmation of Preventive Effect Against Virus

Specific pathogen-free (SPF) female C57BL/6 mice (7 weeks old, Samtako, Korea) were used. The animals were bred under conditions of a temperature of 23±3 °C and a relative humidity of 55±15%, with a 12-hour light/dark cycle, and each test group was organized such that the average body weight was uniformly distributed.

In addition, the influenza A H1N1 virus was inoculated into embryonated fertilized eggs, and the culture fluid was collected at 48 hours, after which the collected virus was quantified and the LD50 was calculated. The prepared virus solution was diluted to 1 LD50, and 30 µL of the diluted solution was used.

Using the above animal model, the constitution of each test group and the setting of the administration dose of each test substance were as shown in Table 2 below. G1 was the normal control group, and G2 was the infection control group; Comparative Examples 1 to 3 corresponded to groups G3 to G5, respectively, and the respective substances of Examples 1 to 4 corresponded to groups G6 to G9. Administration of each substance was started 2 hours before viral infection, and the substances were administered twice daily (bis in die, BID) at 8-hour intervals for 7 days.

Comparative Example 2 was administered orally (per oral, P.O.), whereas the remaining Comparative Examples 1 and 3 and Examples 1 to 4 were administered intranasally (intranasal administration, I.N.). The test animals were restrained using the dorsal skin fixation method, and in the case of Comparative Example 2, the test substance was directly administered into the stomach using an oral gavage sonde. In the case of Examples 1, 2, and 4, the test substance was administered into the nasal cavity by spraying using a spray, and in the case of Example 3, the test substance was well shaken before administration to form a suspension and then administered into the nasal cavity by instillation using a micropipette.

**[Table 2]**

| Gro up | No. of animals | Infec tion | Test substance | No. of admini stratio ns | Route of administr ation | Dose per administrat ion (mg/head) | Volume per administrat ion (µL/head) | Time of administrat ion |
|---|---|---|---|---|---|---|---|---|
| G1 | 4 | N | - | - | - | - | - | Administra tion started 2 hours before infection |
| G2 | 15 | Y | - | - | - | - | - | |
| G3 | 15 | Y | Comparative Example 1 | BID | I.N | - | 20 | and was performed twice daily (BID) at 8-hour intervals for 7 days. |
| G4 | 15 | Y | Comparative Example 2 | BID | P.O | 0.1 | 20 | |
| G5 | 15 | Y | Comparative Example 3 | BID | I.N | 0.024 | 20 | |
| G6 | 15 | Y | Example 1 | BID | I.N | 0.092 | 20 | |
| G7 | 15 | Y | Example 2 | BID | I.N | 0.002 | 20 | |
| G8 | 15 | Y | Example 3 | BID | I.N | 0.18 | 20 | |
| G9 | 15 | Y | Example 4 | BID | I.N | 0.16 | 20 | |

### 1-1. Measurement of Body Weight

The body weight of each animal was measured once daily using an electronic balance. As a result of the measurement, as shown in Table 3 and FIG. 1 below, groups G4 and G5, to which the positive control substances were administered, and groups G6 to G9, to which the respective test substances of Examples 1 to 4 were administered, showed significantly higher levels after 3 DPI (3 days post infection) as compared with the control group G3.

When infected with influenza virus, excessive cellular infiltrates occur in the airway and inflammatory mediators such as IFN are produced, resulting in a decrease in body weight. When the test substances of Examples 1 to 4 of the present invention were administered, it was confirmed that the decrease in body weight, which is one of the symptoms of influenza infection, was alleviated.

**[Table 3]**

| Body weight | | | |
|---|---|---|---|
| UNIT: % to 0 DPI(Mean ± S.D) | | | |
| DPI | Test group | | |
| | G1 | G2 | G3 |
| 0 | 100.00 ± 0.00 | 100.00 ± 0.00 | 100.00 ± 0.00 |
| 1 | 101.35 ± 0.69 | 96.80 ± 1.76*** | 96.30 ± 1.85*** |
| 2 | 104.87 ± 0.91 | 98.72 ± 1.98*** | 95.66 ± 2.44***^{,###} |
| 3 | 105.28 ± 1.43 | 97.36 ± 2.71*** | 93.85 ± 2.81***^{,##} |
| 4 | 103.97 ± 0.62 | 97.57 ± 2.44** | 91.83 ± 3.10***^{,###} |
| 5 | 104.90 ± 2.33 | 96.17 ± 2.37*** | 92.25 ± 3.31*** |
| 6 | - | 94.44 ± 0.94 | 90.53 ± 3.16 |
| 7 | - | 93.44 ± 1.31 | 88.49 ± 3.25 |

| DPI | Test group | | |
|---|---|---|---|
| | G4 | G5 | G6 |
| 0 | 100.00 ± 0.00 | 100.00 ± 0.00 | 100.00 ± 0.00 |
| 1 | 96.86 ± 1.40*** | 96.76 ± 1.52*** | 97.02 ± 1.63*** |
| 2 | 98.36 ± 2.41***^{,$} | 96.37 ± 2.08***^{,#} | 96.82 ± 1.67*** |
| 3 | 98.71 ± 3.01***^{,$$$} | 96.76 ± 2.08***^{,$} | 97.14 ± 1.63***^{,$$} |
| 4 | 100.15 ± 2.84^{$$$} | 96.34 ± 2.52***^{,$$} | 96.15 ± 3.00***^{,$$} |
| 5 | 99.52 ± 2.15*^{,#,$$$} | 98.20 ± 3.02**^{,$$$} | 97.10 ± 1.79***^{,$$} |
| 6 | 97.56 ± 1.98^{$$} | 97.89 ± 2.26^{$$} | 99.52 ± 3.68^{$$$} |
| 7 | 94.73 ± 4.14^{$} | 98.58 ± 2.70^{$$$} | 99.14 ± 3.76^{$$$} |

| DPI | Test group | | |
|---|---|---|---|
| | G7 | G8 | G9 |
| 0 | 100.00 ± 0.00 | 100.00 ± 0.00 | 100.00 ± 0.00 |
| 1 | 96.64 ± 2.34*** | 98.02 ± 1.51** | 97.10 ± 2.42*** |
| 2 | 95.73 ± 2.88***^{,##} | 99.14 ± 2.47***^{,$$$} | 97.04 ± 2.45*** |
| 3 | 96.56 ± 2.92***^{,$} | 100.14 ± 2.76**^{,#,$$$} | 97.89 ± 2.92***^{,$$$} |
| 4 | 95.29 ± 3.10***^{,$} | 99.68 ± 3.36^{$$$} | 97.68 ± 2.64**^{,$$$} |
| 5 | 98.03 ± 3.14***^{,$$$} | 99.50 ± 3.75*^{,$$$} | 99.60 ± 3.02*^{,$$$} |
| 6 | 100.14 ± 3.42^{#,$$$} | 101.13 ± 4.00^{##,$$$} | 99.78 ± 2.93^{#,$$$} |
| 7 | 99.49 ± 3.60^{#,$$$} | 97.97 ± 4.32^{$$$} | 98.26 ± 3.60^{$$$} |
| DPI: days post-infection | | | |
| ***/**/* A significant difference at p<0.001/p<0.01/p<0.05 level compared to the G1 | | | |
| ##/# A significant difference at p<0.01/p<0.05 level compared to the G2 | | | |
| $$$/$ A significant difference at p<0.001/p<0.05 level compared to the G3 | | | |

### 1-2. Histopathological Examination

The effect of administration of the test substances of the present invention on inflammation and tissue damage induced by influenza was evaluated in C57BL/6 mice infected with influenza A virus (H1N1) at a titer of 1 LD50. The viral infection study design and test groups were as shown in Table 2 above. Except for G1, animals in the remaining test groups were euthanized and necropsied, five animals per group per day, on days 3, 5, and 7 from the day of virus inoculation. In the case of G1, all animals were euthanized and necropsied on day 5 from the day of virus inoculation. Animals other than those scheduled for necropsy on the designated days were observed until day 7 from the day of virus inoculation.

At necropsy, the nasal cavity was swabbed using a cotton swab moistened with PBS, and the swab was stored frozen at -70 °C until analysis for measurement of viral titer. The excised lungs were divided into two portions so as to include the lesion sites, and the portion for histopathological examination was fixed in 10% neutral buffered formalin (NBF), while the portion for measurement of viral titer was stored frozen at -70 °C until analysis.

Tissues fixed in 10% NBF were subjected to general tissue processing procedures such as trimming, dehydration, paraffin embedding, and sectioning to prepare specimens for histopathological examination, followed by Hematoxylin & Eosin (H&E) staining, and histopathological changes were observed using a light microscope (Olympus BX53, Japan). Lesions in the lungs were observed and evaluated by scoring according to the degree of inflammatory cell infiltration, and the criteria were as shown in Table 4 below.

**[Table 4]**

| **Score** | **Criterion** |
|---|---|
| 0 | No lesions and no inflammatory cell infiltration observed (None) |
| 1 | Inflammatory cells are scattered within the parenchyma (Minimal) |
| 2 | Inflammatory cells are aggregated in <1/3 of the parenchyma (Mild) |
| 3 | Inflammatory cells are aggregated in 1/3 to 2/3 of the parenchyma (Moderate) |
| 4 | Inflammatory cells are aggregated in >2/3 of the parenchyma (Severe) |

As a result, as shown in Table 5 and FIG. 2 below, on day 3 post infection (3 DPI), the scores of G4 and G7 were significantly lower than those of G2 and G3, and it was confirmed that, up to day 7 post infection, the scores of groups G4 and G5, to which the positive control substances were administered, and groups G6 to G9, to which the respective test substances of Examples 1 to 4 were administered, were lower than those of G2 and G3.

**[Table 5]**

| Histopathological Examination | | | |
|---|---|---|---|
| UNIT: Score | | | |
| DPI | Test group | | |
| | G1 | G2 | G3 |
| 3 | - | 1.4 ± 0.5 | 1.2 ± 0.4 |
| 5 | 0.0 ± 0.0 | 1.8 ± 0.8 | 2.2 ± 0.4 |
| 7 | - | 3.4 ± 0.5 | 3.0 ± 0.0 |

| DPI | Test group | | |
|---|---|---|---|
| | G4 | G5 | G6 |
| 3 | 0.2 ± 0.4^{#,$} | 0.6 ± 0.5 | 0.6 ± 0.5 |
| 5 | 1.0 ± 1.0 | 1.4 ± 0.9 | 1.6 ± 1.1 |
| 7 | 1.4 ± 0.9^{##,$$} | 2.0 ± 1.2 | 2.2 ± 0.8 |
| DPI | Test group | | |

| | G7 | G8 | G9 |
|---|---|---|---|
| 3 | 0.0 ± 0.0^{#,$} | 0.6 ± 0.5 | 0.6 ± 0.5 |
| 5 | 1.2 ± 0.8 | 1.2 ± 1.1 | 1.4 ± 1.1 |
| 7 | 1.4 ± 0.9^{#,$} | 2.2 ± 1.3 | 1.2 ± 0.8^{##,$$} |
| DPI: days post-infection | | | |
| ##/# A significant difference at p<0.01/p<0.05 level compared to the G2 | | | |
| $$/$ A significant difference at p<0.01/p<0.05 level compared to the G3 | | | |

These results indicate that, when the test substances of Examples 1 to 4 of the present invention are administered for prophylactic purposes, lung tissue damage and lesion formation caused by influenza virus can be reduced.

### 1-3. Measurement of Viral Titer

The amount of virus present in lung tissue collected on day 5 after viral infection was quantified by real-time qRT-PCR. A certain amount of WizolTM (WizbioSolution, Seongnam, Korea) reagent was added to the collected lung tissue, the tissue was homogenized using tissue grinding beads, and total RNA was extracted from the supernatant. The extracted total RNA was quantified using a Nanodrop (NanoDrop2000, Thermo Scientific). Using 1 µg of total RNA, cDNA was synthesized with a WizScript^{™} cDNA synthesis kit (WizbioSolution), and an appropriate amount of the cDNA reaction product was then subjected to real-time qPCR using WizPureTM qPCR Master-UDG (WizbioSolution) reagent in accordance with the manufacturer's instructions. Viral genes present in the lung tissue were quantified using primers and probes for M2 (matrix protein 2) and pol PA (polymerase PA), which are specific for influenza virus (A/Puerto Rico/8/1934 (H1N1)). The amount of viral RNA present in the lung tissue was expressed as the number of copies of the viral gene per 1 ng of total RNA. The sequences of each primer and probe are shown in Table 6 below.

**[Table 6]**

| **Gene** | **Type** | **Direction** | **Sequence (5'-3')** | **SEQ ID No.** |
|---|---|---|---|---|
| M2 | M primer | Forward | CTTCTAACCGAGGTCGAAACGTA | 1 |
| | M primer | Reverse | GGTGACAGGATTGGTCTTGTC TTTTA | 2 |
| | M probe | - | [FAM]TCAGGCCCCCTCAAAGCCGAG[BHQ1] | 3 |
| Pol PA | PR8-PA primer | Forward | CGGTCCAAATTCCTGCTGA | 4 |
| | PR8-PA primer | Reverse | CATTGGGTTTCCTTCCATCCA | 5 |
| | PR8-PA probe | - | [HEX]CCAAGTCATGAAGGGAGAGGGGAATACCGCT [BHQ1] | 6 |

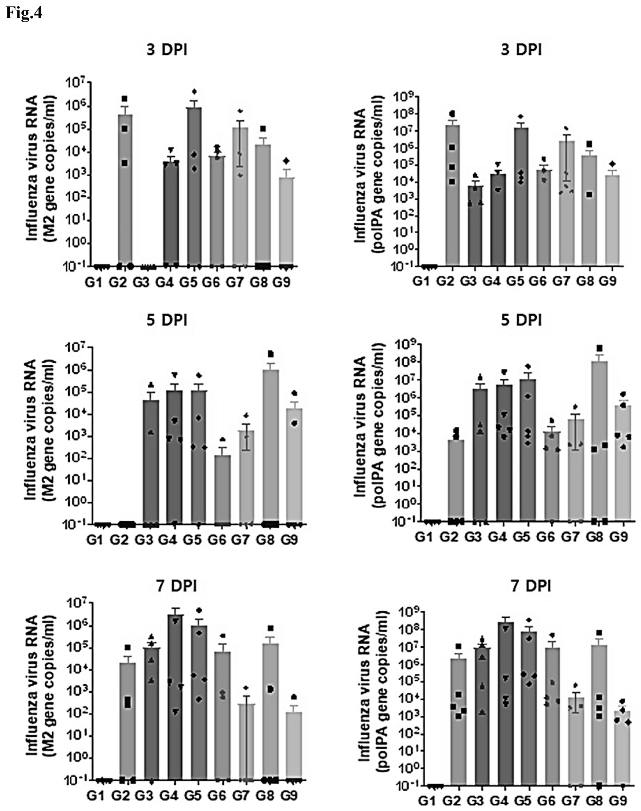

Thereafter, a standard curve was prepared, and the remaining amount of virus in the samples was quantitatively analyzed by applying each of the above genes to the curve. As a result, as shown in FIG. 3, upon administration of the test substances of Examples 1 to 4 of the present invention, the reduction of the M2 and pol PA genes in the lung was not pronounced. In contrast, in the nasal cavity, upon administration of the test substances of Examples 1 to 4 of the present invention, a decrease in the M2 and pol PA genes was observed, as shown in FIG. 4, indicating that there was a preventive effect on influenza virus infection via the nasal cavity. In particular, it was confirmed that a more pronounced effect of reducing viral titer was observed in groups G6, G7, and G9, to which Examples 1, 2, and 4 were administered.

### Experimental Example 2. Confirmation of Therapeutic Effect Against Virus

An animal model was constructed and infected by administration of influenza A H1N1 virus in the same manner as in Experimental Example 1. However, for confirmation of the therapeutic effect, the test substances were administered after viral infection.

Specifically, the constitution of each test group and the setting of the administration dose of each test substance were as shown in Table 7 below. G1 was the normal control group, and G2 was the infection control group; Comparative Examples 1 to 3 corresponded to groups G3 to G5, respectively, and the respective substances of Examples 1 to 4 corresponded to groups G6 to G9. Administration of each substance was started 24 hours after viral infection, and the substances were administered twice daily (bis in die, BID) at 8-hour intervals for 6 days. The methods of administration according to the administration route were the same as in Experimental Example 1.

**[Table 7]**

| Gro up | No. of animals | Infec tion | Test substance | No. of administ rations | Route of administr ation | Dose per administrat ion (mg/head) | Volume per administrat ion (µL/head) | Time of administrat ion |
|---|---|---|---|---|---|---|---|---|
| G1 | 4 | N | - | - | - | - | - | Administra tion started 24 hours after infection and was performed twice daily (BID) at 8-hour intervals for 6 days. |
| G2 | 15 | Y | - | - | - | - | - | |
| G3 | 15 | Y | Comparativ e Example 1 | BID | I.N | - | 20 | |
| G4 | 15 | Y | Comparativ e Example 2 | BID | P.O | 0.1 | 20 | |
| G5 | 15 | Y | Comparativ e Example 3 | BID | I.N | 0.024 | 20 | |
| G6 | 15 | Y | Example 1 | BID | I.N | 0.092 | 20 | |
| G7 | 15 | Y | Example 2 | BID | I.N | 0.002 | 20 | |
| G8 | 15 | Y | Example 3 | BID | I.N | 0.18 | 20 | |
| G9 | 15 | Y | Example 4 | BID | I.N | 0.16 | 20 | |

### 2-1. Measurement of Body Weight

The body weight of each animal was measured once daily using an electronic balance. As a result of the measurement, as shown in Table 8 and FIG. 5 below, on day 5 post infection (5 DPI), groups G6 to G9, to which the respective test substances of Examples 1 to 4 were administered, showed significantly higher levels as compared with the control group G3.

From these results, it was confirmed that, even when the test substances of Examples 1 to 4 of the present invention were administered after infection, the decrease in body weight, which is one of the symptoms of influenza infection, could be alleviated.

**[Table 8]**

| Body weight | | | |
|---|---|---|---|
| UNIT: % to 0 DPI (Mean ± S.D) | | | |
| DPI | Test group | | |
| | G1 | G2 | G3 |
| 0 | 100.00 ± 0.00 | 100.00 ± 0.00 | 100.00 ± 0.00 |
| 1 | 98.34 ± 1.04 | 98.13 ± 1.53 | 97.03 ± 1.09 |
| 2 | 98.36 ± 1.90 | 95.85 ± 1.56 | 96.23 ± 1.78 |
| 3 | 100.29 ± 3.64 | 96.09 ± 1.85 | 95.47 ± 3.43 |
| 4 | 101.28 ± 2.05 | 97.87 ± 1.82 | 94.88 ± 2.51* |
| 5 | 104.59 ± 1.76 | 97.33 ± 3.21* | 94.01 ± 2.90*** |
| 6 | - | 95.11 ± 2.43** | 93.12 ± 1.76*** |
| 7 | - | 92.53 ± 2.73** | 89.33 ± 6.35*** |

| DPI | Test group | | |
|---|---|---|---|
| | G4 | G5 | G6 |
| 0 | 100.00 ± 0.00 | 100.00 ± 0.00 | 100.00 ± 0.00 |
| 1 | 96.35 ± 1.80 | 97.40 ± 1.65 | 97.64 ± 3.47 |
| 2 | 95.82 ± 3.03 | 96.90 ± 2.55 | 96.74 ± 4.87 |
| 3 | 95.76 ± 4.77 | 97.10 ± 3.64 | 96.69 ± 4.92 |
| 4 | 93.96 ± 2.64* | 96.40 ± 3.84 | 96.06 ± 5.84 |
| 5 | 94.75 ± 3.47** | 96.57 ± 4.94* | 95.41 ± 5.53** |
| 6 | 91.73 ± 1.66*** | 97.13 ± 0.95* | 90.70 ± 5.36*** |
| 7 | 90.01 ± 3.20** | 93.39 ± 4.26* | 88.52 ± 6.81*** |

| DPI | Test group | | |
|---|---|---|---|
| | G7 | G8 | G9 |
| 0 | 100.00 ± 0.00 | 100.00 ± 0.00 | 100.00 ± 0.00 |
| 1 | 96.87 ± 2.60 | 97.94 ± 2.27 | 97.64 ± 3.47 |
| 2 | 96.33 ± 3.46 | 96.47 ± 2.00 | 96.74 ± 4.87 |
| 3 | 96.97 ± 3.09 | 96.54 ± 3.60 | 96.69 ± 4.92 |
| 4 | 96.51 ± 4.23 | 95.11 ± 3.76* | 96.06 ± 5.84 |
| 5 | 97.53 ± 3.69* | 94.31 ± 4.23*** | 95.41 ± 5.53** |
| 6 | 96.58 ± 4.64* | 94.61 ± 5.34** | 90.70 ± 5.36*** |
| 7 | 93.85 ± 6.41* | 92.33 ± 6.37** | 88.52 ± 6.81*** |
| DPI: days post-infection | | | |
| ***/**/* A significant difference at p<0.001/p<0.01/p<0.05 level compared to the G1 | | | |

### 2-2. Histopathological Examination

In the same manner as in 1-2 of Experimental Example 1, five animals per group per day were euthanized and necropsied on days 3, 5, and 7 from the day of virus inoculation, and in the case of G1, all animals were euthanized and necropsied on day 5 from the day of virus inoculation, and histopathology was examined. Thereafter, evaluation was performed according to the same scoring criteria as in Table 4 above.

As a result, as shown in Table 9 and FIG. 6 below, at all necropsy time points, the degree of inflammatory cell infiltration in groups G6 to G9, to which the respective test substances of Examples 1 to 4 were administered, tended overall to be lower than that in G3, and on days 5 (5 DPI) and 7 (7 DPI) post infection, the scores of G4, G7, and G9 were significantly lower than those of G2 and G3. In particular, it was confirmed that the degree of inflammatory cell infiltration in G7 and G9 was markedly low.

**[Table 9]**

| Histopathological Examination | | | |
|---|---|---|---|
| UNIT: Score | | | |
| DPI | Test group | | |
| | G1 | G2 | G3 |
| 3 | - | 1.6 ± 1.1 | 1.6 ± 0.5 |
| 5 | 0.0 ± 0.0 | 2.8 ± 0.4 | 2.8 ± 0.4 |
| 7 | - | 3.6 ± 0.5 | 3.2 ± 0.4 |

| DPI | Test group | | |
|---|---|---|---|
| | G4 | G5 | G6 |
| 3 | 0.8 ± 0.4 | 1.0 ± 0.7 | 1.0 ± 0.7 |
| 5 | 1.4 ± 0.5^{#,$} | 2.0 ± 1.0 | 1.8 ± 0.8 |
| 7 | 1.8 ± 0.8^{#,$} | 2.6 ± 0.9 | 2.4 ± 1.3 |

| DPI | Test group | | |
|---|---|---|---|
| | G7 | G8 | G9 |
| 3 | 1.2 ± 0.4 | 1.0 ± 0.7 | 1.0 ± 0.7 |
| 5 | 1.4 ± 0.9^{#,$} | 2.0 ± 1.0 | 1.2 ± 0.8^{#,$} |
| 7 | 1.6 ± 0.5^{##,$$} | 2.4 ± 1.3 | 1.6 ± 0.5^{##,$$} |
| DPI: days post-infection | | | |
| ##/# A significant difference at p<0.01/p<0.05 level compared to the G2 | | | |
| $$/$ A significant difference at p<0.01/p<0.05 level compared to the G3 | | | |

These results indicate that, even when the test substances of Examples 1 to 4 of the present invention are administered after influenza virus infection, lung tissue damage and lesion formation caused by influenza virus can be reduced, in that they demonstrate a therapeutic effect against influenza virus.

### 2-3. Measurement of Viral Titer

In the same manner as in 1-3 of Experimental Example 1, RNA was extracted from lung and nasal cavity swab samples, and the remaining amount of virus was quantitatively analyzed by detecting the M2 and pol PA genes through qRT-PCR.

As a result, as shown in Tables 10 and 11 and FIG. 7 below, in the lung tissues of animals necropsied at 7 DPI, it was confirmed that, as compared with the G3 control group, the M2 and pol PA genes were both decreased in the G5 positive control group and in the test substance-administered groups G7 and G9. In addition, in the case of group G6, it was confirmed that, in the lung tissues of animals necropsied at 5 DPI, the M2 and pol PA genes were both decreased as compared with the G3 control group.

**[Table 10]**

| Viral Titer (Lung) | | | |
|---|---|---|---|
| M2 UNIT: copies/ng | | | |
| DPI | Test group | | |
| | G1 | G2 | G3 |
| 3 | - | 78.08 ± 102.34 | 27.33 ± 30.14 |
| 5 | 0.00 ± 0.00 | 37.48 ± 34.71 | 115.52 ± 117.42 |
| 7 | - | 11.46 ± 9.89 | 4.61 ± 4.87 |

| DPI | Test group | | |
|---|---|---|---|
| | G4 | G5 | G6 |
| 3 | 35.02 ± 60.34 | 25.57 ± 26.40 | 13.90 ± 11.61 |
| 5 | 8.98 ± 10.54 | 107.90 ± 176.40 | 35.34 ± 49.38 |
| 7 | 8.86 ± 7.64 | 4.53 ± 6.94 | 6.38 ± 4.72 |

| DPI | Test group | | |
|---|---|---|---|
| | G7 | G8 | G9 |
| 3 | 6.61 ± 9.18 | 14.52 ± 22.43 | 30.11 ± 25.51 |
| 5 | 43.52 ± 88.41 | 54.11 ± 99.04 | 126.93 ± 155.92 |
| 7 | 3.18 ± 3.12 | 11.40 ± 12.05 | 2.41 ± 2.38 |

**[Table 11]**

| Viral Titer (Lung) | | | |
|---|---|---|---|
| pol PA UNIT: copies/ng | | | |
| DPI | Test group | | |
| | G1 | G2 | G3 |
| 3 | - | 2715.87 ± 3666.96 | 1009.60 ± 1413.18 |
| 5 | 0.00 ± 0.00 | 491.84 ± 540.21 | 1242.72 ± 1200.10 |
| 7 | - | 174.77 ± 228.20 | 63.05 ± 63.85 |

| DPI | Test group | | |
|---|---|---|---|
| | G4 | G5 | G6 |
| 3 | 1453.46 ± 2714.18 | 645.15 ± 794.64 | 432.91 ± 335.67 |
| 5 | 132.86 ± 104.09 | 2282.70 ± 4021.43 | 465.80 ± 648.05 |
| 7 | 165.93 ± 182.61 | 53.94 ± 79.55 | 76.27 ± 50.37 |

| DPI | Test group | | |
|---|---|---|---|
| | G7 | G8 | G9 |
| 3 | 54.76 ± 77.74 | 407.23 ± 767.51 | 1050.61 ± 914.06 |
| 5 | 1223.43 ± 1798.12 | 472.83 ± 780.71 | 1462.30 ± 1905.12 |
| 7 | 47.74 ± 55.54 | 176.85 ± 152.72 | 29.74 ± 34.23 |

In addition, as shown in Tables 12 and 13 and FIG. 8 below, in the nasal cavity samples from animals necropsied at 7 DPI, it was confirmed that, as compared with the G3 control group, the M2 and pol PA genes were both decreased in the G5 positive control group and in the test substance-administered groups G6, G7, and G9.

**[Table 12]**

| Viral Titer (Nasal cavity) | | | |
|---|---|---|---|
| M2 UNIT: copies/mL | | | |
| DPI | Test group | | |
| | G1 | G2 | G3 |
| 3 | - | 720.63 ± 1188.14 | 0.00 ± 0.00 |
| 5 | 0.00 ± 0.00 | 865847.04 ± 1930483.29 | 825281.04 ± 1845384.51 |
| 7 | - | 30780.88 ± 66589.93 | 28537.64 ± 63675.62 |

| DPI | Test group | | |
|---|---|---|---|
| | G4 | G5 | G6 |
| 3 | 0.00 ± 0.00 | 327630.74 ± 698396.61 | 399.55 ± 893.43 |
| 5 | 67612.73 ± 117098.05 | 8039152.61 ± 10368970.73 | 5387.49 ± 11770.65 |
| 7 | 264852.85 ± 236609.52 | 515.45 ± 666.50 | 11598.79 ± 18410.41 |

| DPI | Test group | | |
|---|---|---|---|
| | G7 | G8 | G9 |
| 3 | 96744.31 ± 208185.08 | 359.83 ± 804.59 | 3470.91 ± 6150.44 |
| 5 | 137.75 ± 252.03 | 60885.81 ± 133470.76 | 599.00 ± 1339.41 |
| 7 | 14499.47 ± 32421.80 | 39763.68 ± 87024.28 | 3305.15 ± 7390.53 |

**[Table 13]**

| Viral Titer (Nasal cavity) | | | |
|---|---|---|---|
| pol PA UNIT: copies/mL | | | |
| DPI | Test group | | |
| | G1 | G2 | G3 |
| 3 | - | 8173.31 ± 7070.26 | 10521.46 ± 19578.47 |
| 5 | 0.00 ± 0.00 | 42551552.83 ± 95009881.85 | 586.32 ± 1077.78 |
| 7 | - | 4738095.80 ± 10514434.11 | 3314005.76 ± 7390510.19 |

| DPI | Test group | | |
|---|---|---|---|
| | G4 | G5 | G6 |
| 3 | 687.83 ± 832.96 | 18236216.91 ± 38691441.18 | 11313.29 ± 18427.97 |
| 5 | 1905385.64 ± 2935251.54 | 411088263.44 ± 488467822.79 | 105133.35 ± 226762.09 |
| 7 | 26587812.07 ± 26169008.36 | 49594.24 ± 83919.74 | 1523582.19 ± 2434770.92 |

| DPI | Test group | | |
|---|---|---|---|
| | G7 | G8 | G9 |
| 3 | 5222876.41 ± 11628560.11 | 11808.16 ± 21575.74 | 107941.59 ± 196643.62 |
| 5 | 5438.77 ± 6900.60 | 3705803.49 ± 8212700.01 | 12887.58 ± 22429.61 |
| 7 | 2069412.39 ± 4607026.07 | 3269283.91 ± 7276365.49 | 430285.99 ± 960128.44 |

From the above results, it was confirmed that, upon administration of the test substances of Examples 1, 2, and 4 of the present invention, the viral titer was reduced, and thus they are effective in the treatment of influenza virus infection.

### Experimental Example 3. Confirmation of Antiviral Effect According to Xanthan Gum Content

In order to confirm the antiviral effect according to the xanthan gum content, an animal model was constructed and influenza A H1N1 virus was administered in the same manner as in Experimental Example 1. The specific constitution of the test groups and the setting of the administration dose of each test substance are shown in Table 14 below.

**[Table 14]**

| Group | No. of animals | Infection | Test substance | No. of administrations | Route of administration | Dose per administration (mg/head) | Volume per administration (µL/head) | Time of administration |
|---|---|---|---|---|---|---|---|---|
| G1 | 4 | N | - | - | - | - | - | Administration started 2 hours before infection |
| G2 | 18 | Y | - | - | - | - | - | |
| G3 | 18 | Y | Comparative Example 1 | BID | I.N | - | 20 | and was performed twice daily (BID) at 8-hour intervals for 7 days. |
| G4 | 18 | Y | Comparative Example 3 | BID | I.N | 0.024 | 20 | |
| G5 | 18 | Y | Example 5 | BID | I.N | 0.005 | 20 | |
| G6 | 18 | Y | Example 6 | BID | I.N | 0.010 | 20 | |
| G7 | 18 | Y | Example 7 | BID | I.N | 0.020 | 20 | |

### 3-1. Measurement of Body Weight

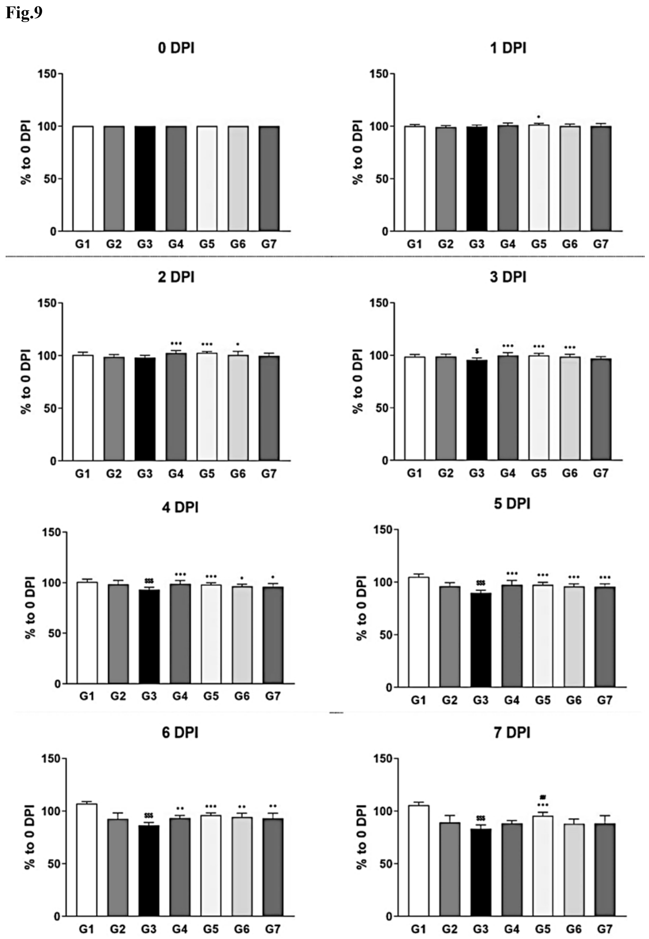

The body weight of each animal was measured once daily using an electronic balance. As a result of the measurement, as shown in Table 15 and FIG. 9 below, all test groups G5 to G7, to which xanthan gum was administered, showed higher levels than group G3. That is, it was confirmed that, even when xanthan gum was administered at a low concentration, a medium concentration, or a high concentration, the decrease in body weight, which is one of the symptoms of influenza infection, could be alleviated.

**[Table 15]**

| Body weight | | | | | | |
|---|---|---|---|---|---|---|
| UNIT: % to 0 DPI(Mean ± S.D) | | | | | | |
| DPI | Test group | | | | | |
| | G1 | G2 | G3 | | G4 | |
| 0 | 100.00 ± 0.00 | 100.00 ± 0.00 | 100.00 ± 0.00 | | 100.00 ± 0.00 | |
| 1 | 100.14 ± 1.48 | 99.18 ± 1.44 | 99.89 ± 1.33 | | 100.99 ± 2.07 | |
| 2 | 100.70 ± 2.44 | 98.79 ± 2.08 | 98.24 ± 2.09 | | 102.38 ± 2.29*** | |
| 3 | 98.60 ± 2.27 | 98.90 ± 2.31 | 95.56 ± 2.06^{$} | | 99.78 ± 2.72*** | |
| 4 | 100.98 ± 2.57 | 98.40 ± 3.86 | 93.18 ± 2.27^{$$$} | | 98.87 ± 3.37*** | |
| 5 | 104.77 ± 2.92 | 96.15 ± 3.24 | 89.82 ± 2.52^{$$$} | | 97.32 ± 4.29*** | |
| 6 | 107.01 ± 2.06 | 92.53 ± 5.61 | | 86.51 ± 2.70^{$$$} | | 93.43 ± 2.42** |
| 7 | 105.75 ± 2.59 | 89.25 ± 6.54 | | 83.29 ± 3.52^{$$$} | | 88.38 ± 2.59 |

| DPI | Test group | | | | | |
|---|---|---|---|---|---|---|
| | G5 | | G6 | | G7 | |
| 0 | 100.00 ± 0.00 | | 100.00 ± 0.00 | | 100.00 ± 0.00 | |
| 1 | 101.62 ± 1.07* | | 100.07 ± 1.83 | | 100.12 ± 2.47 | |
| 2 | 102.60 ± 1.31*** | | 100.58 ± 3.46* | | 99.72 ± 2.58 | |
| 3 | 99.80 ± 2.04*** | | 98.55 ± 2.42*** | | 96.87 ± 1.83 | |
| 4 | 98.01 ± 1.90*** | | 96.48 ± 2.04* | | 95.91 ± 3.27* | |
| 5 | 97.27 ± 2.48*** | | 95.91 ± 2.51*** | | 95.56 ± 2.90*** | |
| 6 | 96.16 ± 1.97*** | | 94.39 ± 3.58** | | 93.18 ± 4.81** | |
| 7 | 95.69 ± 3.07***^{,##} | | 87.97 ± 4.50 | | 88.25 ± 7.49 | |
| DPI: days post-infection | | | | | | |
| $$$/$ A significant difference at p<0.001/p<0.05 level compared to the G1 | | | | | | |
| ***/**/* A significant difference at p<0.001/p<0.01/p<0.05 level compared to the G3 | | | | | | |
| ## A significant difference at p<0.01 compared to the G4 | | | | | | |

### 3-2. Histopathological Examination

In the same manner as in 1-2 of Experimental Example 1, six animals per group per day were euthanized and necropsied on days 3, 5, and 7 from the day of virus inoculation, and in the case of G1, all animals were euthanized and necropsied on day 5 from the day of virus inoculation, and histopathology was examined. Thereafter, evaluation was performed according to the same scoring criteria as in Table 4 above.

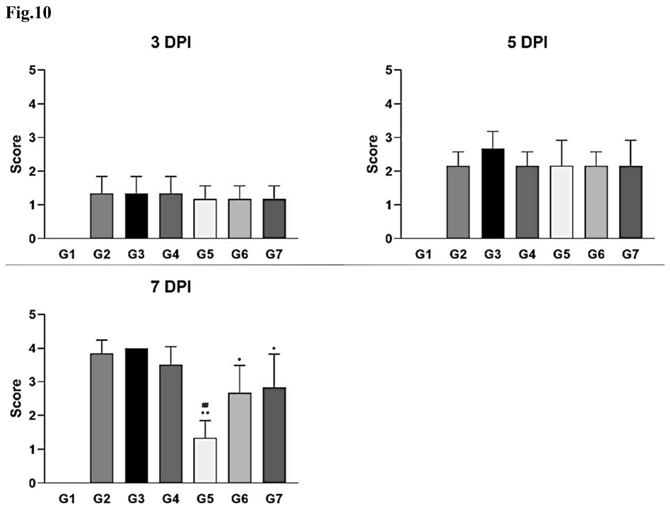

As a result, as shown in Table 16 and FIG. 10 below, at all necropsy time points, the degree of inflammatory cell infiltration in groups G5 to G7, to which the respective test substances of Examples 5 to 7 were administered, was lower than that in G3. In particular, it was confirmed that the degree of inflammatory cell infiltration in G5, to which xanthan gum was administered at a low concentration, was markedly low.

**[Table 16]**

| Histopathological Examination | | | | | | |
|---|---|---|---|---|---|---|
| UNIT: Score | | | | | | |
| DPI | Test group | | | | | |
| | G1 | G2 | | G3 | | G4 |
| 3 | - | 1.3 ± 0.5 | | 1.3 ± 0.5 | | 1.3 ± 0.5 |
| 5 | - | 2.2 ± 0.4 | | 2.7 ± 0.5 | | 2.2 ± 0.4 |
| 7 | 0.0 ± 0.0 | 3.8 ± 0.4 | | 4.0 ± 0.0 | | 3.5 ± 0.5 |

| DPI | Test group | | | | | |
|---|---|---|---|---|---|---|
| | G5 | | G6 | | G7 | |
| 3 | 1.2 ± 0.4 | | 1.2 ± 0.4 | | 1.2 ± 0.4 | |
| 5 | 2.2 ± 0.8 | | 2.2 ± 0.4 | | 2.2 ± 0.8 | |
| 7 | 1.3 ± 0.5**^{,##} | | 2.7 ± 0.8* | | 2.8 ± 1.0* | |
| DPI: days post-infection | | | | | | |
| **/* A significant difference at p<0.01/p<0.05 level compared to the G3 | | | | | | |
| ## A significant difference at p<0.01 compared to the G4 | | | | | | |

These results indicate that, over all concentration ranges of xanthan gum, lung tissue damage and lesion formation caused by influenza virus can be reduced.

### 3-3. Measurement of Viral Titer

In the same manner as in 1-3 of Experimental Example 1, RNA was extracted from lung and nasal cavity swab samples, and the remaining amount of virus was quantitatively analyzed by detecting the M2 and pol PA genes through qRT-PCR.

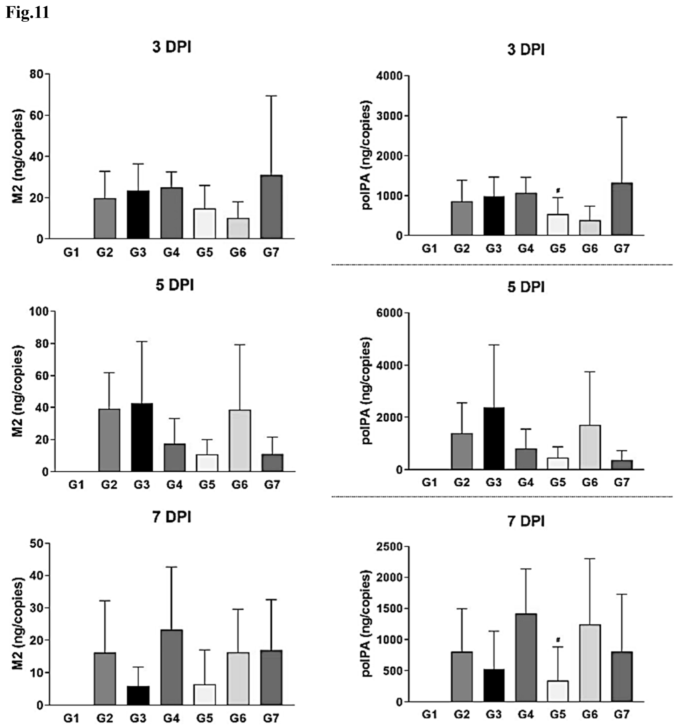

As a result, as shown in Tables 17 and 18 and FIG. 11 below, in the lung tissues of animals necropsied at 3 DPI and 5 DPI, it was confirmed that, as compared with the G3 control group, the M2 and pol PA genes were both decreased in the test substance-administered groups G5 to G7.

**[Table 17]**

| Viral Titer (Lung) | | | | | | |
|---|---|---|---|---|---|---|
| M2 UNIT: copies/ng | | | | | | |
| DPI | Test group | | | | | |
| | G1 | G2 | | G3 | | G4 |
| 3 | - | 19.89 ± 12.97 | | 23.61 ± 12.85 | | 25.13 ± 7.46 |
| 5 | - | 39.19 ± 22.57 | | 42.57 ± 38.62 | | 17.34 ± 15.81 |
| 7 | 0.00 ± 0.00 | 16.13 ± 16.04 | | 5.91 ± 5.82 | | 23.22 ± 19.36 |

| DPI | Test group | | | | | |
|---|---|---|---|---|---|---|
| | G5 | | G6 | | G7 | |
| 3 | 14.73 ± 11.33 | | 10.09 ± 8.02 | | 31.00 ± 38.36 | |
| 5 | 10.86 ± 9.22 | | 38.74 ± 40.44 | | 11.04 ± 10.39 | |
| 7 | 6.45 ± 10.63 | | 16.24 ± 13.31 | | 16.95 + 15.56 | |

**[Table 18]**

| Viral Titer (Lung) | | | | | | |
|---|---|---|---|---|---|---|
| pol PA UNIT: copies/ng | | | | | | |
| DPI | Test group | | | | | |
| | G1 | G2 | | G3 | | G4 |
| 3 | - | 857.54 ± 532.06 | | 989.44 ± 478.52 | | 1070.80 ± 394.54 |
| 5 | - | 1404.37 ± 1147.11 | | 2367.40 ± 2412.48 | | 808.85 ± 742.05 |
| 7 | 0.04 ± 0.07 | 805.97 ± 689.61 | | 530.64 ± 606.43 | | 1414.91 ± 718.32 |

| DPI | Test group | | | | | |
|---|---|---|---|---|---|---|
| | G5 | | G6 | | G7 | |
| 3 | 532.63 ± 426.17* | | 380.05 ± 360.48 | | 1327.49 ± 1632.89 | |
| 5 | 458.61 ± 415.89 | | 1711.80 ± 2046.23 | | 365.94 ± 359.98 | |
| 7 | 340.79 ± 540.49^{#} | | 1241.74 ± 1057.86 | | 808.61 ± 917.61 | |
| # A significant difference at p<0.05 level compared to G4 | | | | | | |

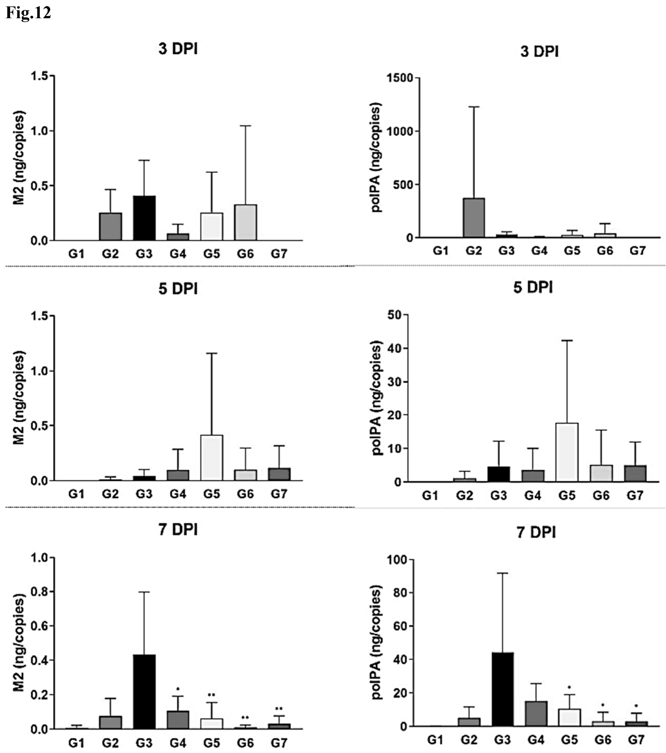

In addition, as shown in Tables 19 and 20 and FIG. 12 below, in the nasal cavity samples from animals necropsied at 7 DPI, it was confirmed that, as compared with the G3 control group, the M2 and pol PA genes were both decreased in the test substance-administered groups G5 to G7.

**[Table 19]**

| Viral Titer (Nasal cavity) | | | | | | |
|---|---|---|---|---|---|---|
| M2 UNIT: copies/ng | | | | | | |
| DPI | Test group | | | | | |
| | G1 | G2 | | G3 | | G4 |
| 3 | - | 0.25 ± 0.21 | | 0.41 ± 0.32 | | 0.64 ± 1.39 |
| 5 | - | 0.01 ± 0.03 | | 0.04 ± 0.06 | | 0.10 ± 0.19 |
| 7 | 0.01 ± 0.02 | 0.07 ± 0.10 | | 0.43 ± 0.36 | | 0.11 ± 0.09* |

| DPI | Test group | | | | | |
|---|---|---|---|---|---|---|
| | G5 | | G6 | | G7 | |
| 3 | 0.25 ± 0.37 | | 0.33 ± 0.71 | | 0.00 ± 0.00 | |
| 5 | 0.42 ± 0.74 | | 0.10 ± 0.20 | | 0.12 ± 0.20 | |
| 7 | 0.06 ± 0.09** | | 0.01 ± 0.01** | | 0.03 ± 0.05** | |
| **/* A significant difference at p<0.01/p<0.05 level compared to the G3 | | | | | | |

**[Table 20]**

| Viral Titer (Nasal cavity) | | | | | | |
|---|---|---|---|---|---|---|
| pol PA UNIT: copies/ng | | | | | | |
| DPI | Test group | | | | | |
| | G1 | G2 | | G3 | | G4 |
| 3 | - | 374.78 ± 853.33 | | 29.90 ± 26.57 | | 20.80 ± 40.74 |
| 5 | - | 1.08 ± 2.11 | | 4.58 ± 7.60 | | 3.59 ± 6.42 |
| 7 | 0.04 ± 0.07 | 4.97 ± 6.68 | | 44.23 ± 47.42 | | 15.09 ± 10.57 |

| DPI | Test group | | | | | |
|---|---|---|---|---|---|---|
| | G5 | | G6 | | G7 | |
| 3 | 24.33 ± 43.38 | | 39.47 ± 92.81 | | 0.06 ± 0.09 | |
| 5 | 17.64 ± 24.67 | | 5.16 ± 10.37 | | 4.96 ± 7.01 | |
| 7 | 10.53 ± 8.46* | | 2.97 ± 5.49* | | 3.04 ± 4.80* | |
| * A significant difference at p<0.05 level compared to G3 | | | | | | |

From the above results, it was confirmed that, over all concentration ranges of xanthan gum, the titer of influenza virus was reduced, and thus it is effective in the prevention and/or treatment of influenza virus infection.

### Experimental Example 4. Statistical Analysis

The results of each of the above Experimental Examples were analyzed on the assumption of normality of the data, using parametric multiple comparison procedures or non-parametric multiple comparison procedures.

In the case of parametric multiple comparisons, normality of the data was assumed and the data were tested by parametric one-way analysis of variance (one-way ANOVA), and when the results were significant, Dunnett's multiple comparison test was used for post hoc analysis to analyze significant differences between test groups. In the case of non-parametric multiple comparisons, the data were tested by Kruskal-Wallis' H-test, and when the results were significant, Mann-Whitney U test, as a post hoc analysis, was used to analyze significant differences between test groups. Statistical analysis was performed using Prism 9.4.1 (GraphPad Software Inc., San Diego, CA, USA), and when the p-value was less than 0.05, the result was determined to be statistically significant.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive. For example, each constituent element which is described as a singular form may be implemented in a distributed form, and similarly, constituent elements which are described as being distributed may be implemented in a combined form.

The scope of the present invention is represented by the claims to be described below, and it should be interpreted that the meaning and scope of the claims and all the changes or modified forms derived from the equivalent concepts thereof fall within the scope of the present invention.

## Claims

1. An antiviral composition against influenza virus, comprising xanthan gum or fucoidan as an active ingredient.

2. The antiviral composition of claim 1, wherein the influenza virus is an influenza A virus.

3. The antiviral composition of claim 2, wherein the influenza A virus is of the H1N1 type.

4. The antiviral composition of claim 1, wherein the composition is for immunization against influenza virus infection.

5. A pharmaceutical composition for the prevention or treatment of a viral infectious disease caused by influenza virus, comprising xanthan gum or fucoidan as an active ingredient.

6. The pharmaceutical composition of claim 5, wherein the viral infectious disease caused by influenza virus is selected from the group consisting of a cold, influenza, sore throat, laryngitis, pharyngitis, rhinitis, bronchitis, asthma, sepsis and pneumonia.

7. The pharmaceutical composition of claim 5, wherein the pharmaceutical composition is for non-oral administration.

8. The pharmaceutical composition of claim 7, wherein the non-oral administration is intranasal or into the nasal cavity.

9. The pharmaceutical composition of claim 8, wherein the intranasal or nasal cavity administration is carried out in the form of a spray, aerosol or inhalation.

10. A method for treating influenza virus infection, comprising administering the pharmaceutical composition of claim 5 to a subject.

11. Use of the pharmaceutical composition of claim 5 for treating influenza virus infection.

12. Use of the antiviral composition of claim 1 for the manufacture of a medicament for treating influenza virus infection.
